# EUROPÄISCHE PATENTSCHRIFT

(11) **EP 0 900 782 B1**
(45) Veröffentlichungstag und Bekanntmachung des Hinweises auf die Patenterteilung: **30.01.2002**
(21) Anmeldenummer: 98120701.2
(22) Anmeldetag: 03.11.1995
(51) Int. Cl.: C07C 255/23, C07C 255/30, C07C 255/41, C09K 15/16

(54) **2-Cyanacrylsäureester**
2-Cyanoacrylic acid esters
Esters de l'acide cyanoacrylique

(30) Priorität: 10.11.1994 DE 4440055; 31.05.1995 DE 19519895
(43) Veröffentlichungstag der Anmeldung: 10.03.1999
(62) Teilanmeldung aus: 95938395.1
(73) Patentinhaber: BASF AKTIENGESELLSCHAFT, 67056 Ludwigshafen (DE)
(72) Erfinder: Holderbaum, Martin, Dr., 67065 Ludwigshafen (DE); Aumüller, Alexander, Dr., 67435 Neustadt (DE); Trauth, Hubert, 67373 Dudenhofen (DE); Voit, Guido, Dr., 69198 Schriesheim (DE); Sperling, Karin, Dr., 67433 Neustadt (DE); Krause, Alfred, Dr., 67346 Speyer (DE)

(56) Entgegenhaltungen:
- PATENT ABSTRACTS OF JAPAN vol. 13, no. 494 (C-651), 8. November 1989 & JP 01 197464 A (MATSUMOTO SEIYAKU KOGYO KK), 9. August 1989
- PATENT ABSTRACTS OF JAPAN vol. 9, no. 287 (C-314), 14. November 1985 & JP 60 133082 A (TOA GOSEI KAGAKU KOGYO KK), 16. Juli 1985
- PATENT ABSTRACTS OF JAPAN vol. 9, no. 267 (C-310), 24. Oktober 1985 & JP 60 115676 A (TOA GOSEI KAGAKU KOGYO KK), 22. Juni 1985
- PATENT ABSTRACTS OF JAPAN vol. 8, no. 122 (C-227), 8. Juni 1984 & JP 59 036177 A (TOA GOSEI KAGAKU KOGYO KK), 28. Februar 1984

## Beschreibung

Die vorliegende Erfindung betrifft neue 2-Cyanacrylsäureester der Formel I, 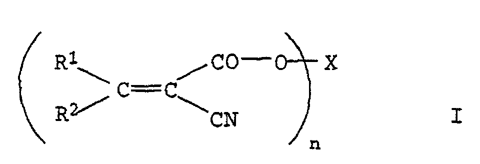 in der die Reste R¹ und R² Wasserstoff oder einen Rest mit einem iso- oder heterocyclischen Ringsystem mit mindestens einem iso - oder heteroaromatischen Kern bedeuten, wobei mindestens einer der Reste R¹ oder R² von Wasserstoff verschieden sein muß,
n einen Wert von > 2 bis 10 hat und
X den Rest eines n-wertigen aliphatischen oder cycloaliphatischen Polyols mit 3 - 20 C-Atomen bezeichnet, wobei ein cycloaliphatischer Rest auch 1 bis 2 Heteroatome enthalten kann und ein aliphatischer Rest durch bis zu 8 nicht benachbarte Sauerstoffatome, Schwefelatome, Imino- oder C₁-C₄-Alkyliminogruppen unterbrochen sein kann.

Weiterhin betrifft die Erfindung ein Verfahren zur Herstellung der Verbindungen I, ihre Verwendung als Lichtschutzmittel oder Stabilisatoren für organische Materialien, insbesondere für kosmetische oder dermatologische Zubereitungen, Kunststoffe oder Lacke sowie organische Materialien, welche die Verbindungen I enthalten.

Aus der US-A 3 215 725 (1) und der DE-A 41 22 475 sind 2-Cyanacrylsäureester von einwertigen und zweiwertigen Alkoholen als Lichtschutzmittel für Kunststoffe und Lacke bekannt.

Diese Verbindungen haben jedoch den anwendungstechnischen Nachteil einer relativ hohen Flüchtigkeit. Da sie außerdem mit vielen organischen Materialien, insbesondere mit Polyolefinen nur bedingt verträglich sind, neigen sie vor allem bei Wärmelagerung zur Migration und darauf beruhenden Ausschwitzeffekten.

Es war daher Aufgabe der Erfindung, diesen Nachteilen durch neue Stabilisatoren vom Typ der 2-Cyanacrylsäureester abzuhelfen.

Demgemäß wurden die eingangs definierten 2-Cyanacrylsäureester der allgemeinen Formel I gefunden.

Weiterhin wurde ein Verfahren zur Herstellung dieser Verbindungen, ihre Verwendung als Lichtschutzfaktoren oder Stabilisatoren von organischen Materialien sowie organische Zubereitungen, die diese Verbindungen als Stabilisatoren enthalten, gefunden.

Wenn die Reste R¹ und R² ungleich sind, können die 2-Cyanacrylsäureestergruppen von I sowohl in der cis- als auch in der trans-Form vorliegen. Bei der Herstellung der Verbindungen entstehen meist Gemische dieser Isomeren. Eine Trennung dieser Isomeren ist möglich, jedoch für die meisten anwendungstechnischen Zwecke nicht erforderlich.

Als organische Reste für R¹ bzw. R² kommen allgemein Ringstrukturen in Betracht, die mindestens einen iso- oder heteroaromatischen Kern enthalten, der vorzugsweise direkt an das 3-C-Atom der Acrylsäuregruppierung gebunden ist, aber auch über aliphatische oder cycloaliphatische Gruppierungen sowie über ein 5 Brückenglied -NR³- mit diesem C-Atom verknüpft sein kann.

Bevorzugt steht R¹ bzw. R² für einen Rest der Formel II 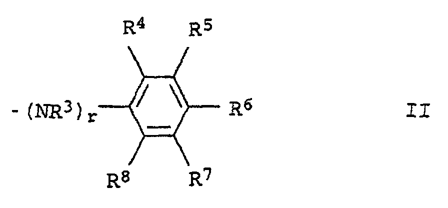 worin R³ Wasserstoff oder C₁-C₁₀-Alkyl bedeutet, r für die Zahl 0 oder 1 steht und R⁴ bis R⁸ unabhängig voneinander Wasserstoff, C₁-C₈-Alkyl, Chlor, Brom, Cyano, Nitro, Amino, Mono(C₁-C₄-alkyl)amino, Di (C₁-C₄ -alkyl)amino, Hydroxy, C₁-C₈-Acyl, C₁-C₈-Acyloxy, C₁-C₁₈-Alkoxy, C₁-C₁₂-Alkoxycarbonyl, C₃-C₆-Cycloalkyl oder C₃-C₆-Cycloalkoxycarbonyl bezeichnen.

Als Reste R³ kommen neben Wasserstoff C₁-C₁₀-Alkylreste in Betracht wie Methyl, Ethyl, n-Propyl, iso-Propyl, iso-Propyl, n-Propyl, n-Butyl, iso-Butyl- sec.-Butyl, tert.-Butyl, n-Pentyl, iso-Pentyl, sec.-Pentyl, tert.-Pentyl, neo-Pentyl, n-Hexyl, n-Heptyl, n-Octyl, iso-Octyl, 2-Ethylhexyl, n-Nonyl, iso-Nonyl, n-Decyl und iso-Decyl.

Sind eine oder mehrere der Reste R⁴ bis R⁸ C₁-C₈-Alkyl, C₁-C₈-Acyl, C₁-C₁₈-Alkoxy oder C₁-C₁₂-Alkoxycarbonyl, so können die darin enthaltenen Alkylreste beispielsweise Methyl, Ethyl, n-Propyl, iso-Propyl, n-Propyl, n-Butyl, iso-Butyl, sec.-Butyl, tert.-Butyl, n-Pentyl, iso-Pentyl, sec.-Pentyl, tert.-Pentyl, neo-Pentyl, n-Hexyl, n-Heptyl, n-Octyl oder 2-Ethylhexyl sein.

Als längerkettige Alkylreste in C₁-C₁₈-Alkoxy bzw. C₁-C₁₂-Alkoxycarbonylgruppen kommen z.B. Nonyl, 2-Methylnonyl, Isononyl, 2-Methyloctyl, Decyl, Isodecyl, 2-Methylnonyl, Undecyl, Isoundecyl, Dodecyl, Isododecyl, Tridecyl, Isotridecyl, Tetradecyl, Pentadecyl, Hexadecyl, Heptadecyl und Octadecyl in Betracht. (Die Bezeichnungen Isooctyl, Isononyl, Isodecyl und Isotridecyl sind Trivialbezeichnungen und stammen von den nach der Oxosynthese erhaltenen Carbonylverbindungen ab; vgl. dazu Ullmann's Encyklopedia of Industrial Chemistry, 5 th Edition, Vol. A1. Seiten 290-293, sowie Vol. A10, Seiten 284 und 285).

Als C₃- bis C₆-Cycloalkylreste eignen sich beispielsweise Cyclopropyl, Cyclobutyl, Cyclopentyl, Methylcyclopentyl oder Cyclohexyl. Diese Cycloalkylgruppen sind auch geeignete Reste in C₃-bis C₆-Cycloalkylcarbonylgruppen.

Bevorzugte 2-Cyanacrylsäureester I sind solche, in denen R³ Wasserstoff, Methyl oder Ethyl bedeutet.

Weiterhin sind solche 2-Cyanacrylsäureester I bevorzugt, in denen bis zu drei, besonders bevorzugt einer der Reste R⁴ bis R⁸ Wasserstoff, C₁-C₄-Alkyl, Chlor, Cyano, Hydroxy, Acetyl, C₁-C₅-Alkoxy, C₁-C₈-Alkoxycarbonyl oder Cyclohexoxycarbonyl und die übrigen dieser Reste Wasserstoff bedeuten.

Besonders bevorzugt sind solche 2-Cyanacrylsäureester I, in denen R⁶ eine Hydroxy-, Methoxy-, Ethoxy-, Propoxy-, Isopropoxy-, Butoxy-, Isobutoxy-, sec-Butoxy- oder tert.-Butoxygruppe bedeutet, da derartige 4-substituierte Phenylgruppen zum stabilisierenden Effekt der Verbindungen beitragen. Aus dem gleichen Grund sind auch solche 2-Cyanacrylsäureester besonders bevorzugt, in denen R⁵ und/oder R⁷ Wasserstoff, eine Methyl- oder tert.-Butylgruppe bedeuten, insbesondere, wenn R⁶ eine Hydroxylgruppe bedeutet.

Unter den erfindungsgemäßen Verbindungen I sind diejenigen bevorzugt, in denen r = O ist.

Weiterhin sind die erfindungsgemäßen Verbindungen bevorzugt, in denen R¹ oder R² Wasserstoff bedeutet, diejenigen, in denen R¹ und R² gleiche Reste sind, sowie diejenigen, in denen einer der Reste R¹ oder R² für Phenylamino, p-Tolylamino, p-Methoxy- oder p-Ethoxycarbonylphenylamino und der andere für Wasserstoff steht.

Ein weiterer bevorzugter Rest für R¹ bzw. R² ist der Chromanrest Ib 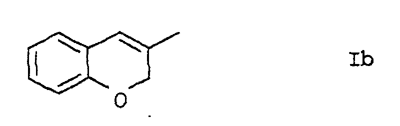 bzw. seine substituierten Derivate, da auch diese die stabilisierende Wirkung der Verbindungen I verstärken.

Als weitere Reste R¹ bzw. R² kommen heterocyclische Gruppen wie substituierte oder unsubstituierte Thiophenyl-, Furfuryl- und Pyridylreste in Betracht.

X steht für den Rest eines n-wertigen aliphatischen oder cycloaliphatischen Alkohols. Diese Alkohole können linear oder verzweigt sein, und ihre C-Ketten können durch ein oder mehrere Sauerstoff- oder Schwefelatome, durch Iminogruppen (-NH-) oder C₁-C₄-Alkyliminogruppen unterbrochen sein.

Die Gruppierung X leitet sich vorzugsweise von folgenden bekannten Polyolen ab:

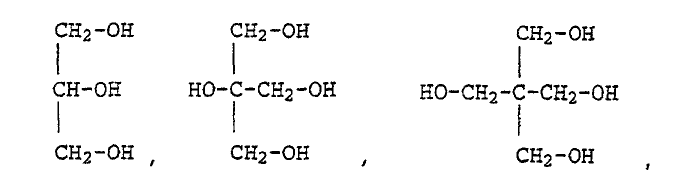

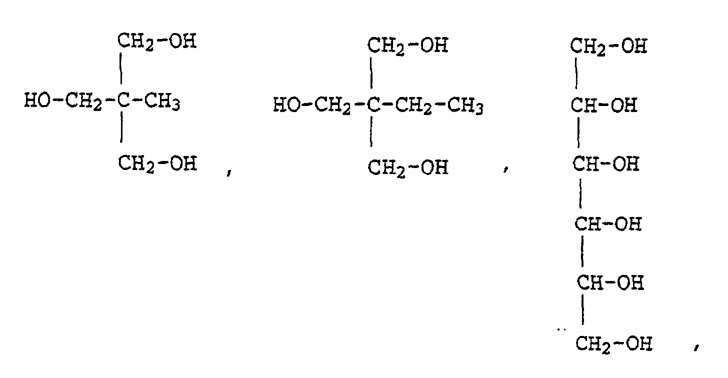

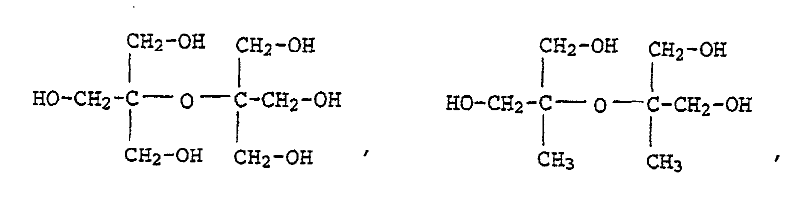

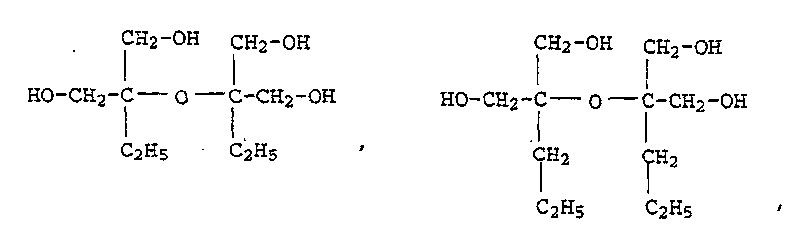

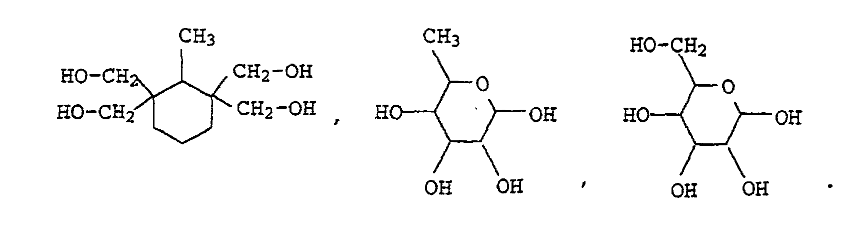

Die 2-Cyanacrylsäureester der Formel I, in denen R¹ und R² nicht über ein Stickstoffatom an das β-C-Atom gebunden sind, sind vorzugsweise durch Umsetzung von Cyanessigsäureestern der allgemeinen Formel III 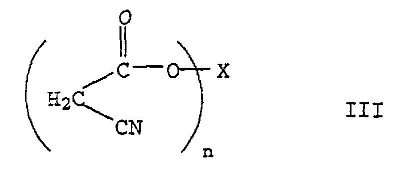 mit n mol einer Verbindung (IV) 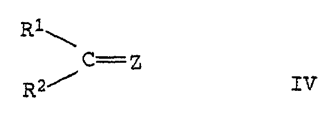 unter den Bedingungen der Knoevenagel-Kondensation erhältlich. Die Umsetzung kann z.B. in aromatischen Lösungsmitteln wie Toluol oder Xylol durchgeführt werden (s. z.B. Organikum, Ausgabe 1976, S. 572). Bevorzugt werden jedoch polare organische Lösungsmittel wie Dimethylformamid, Dimethylacetamid, N-Methylpyrrolidon, Trialkylorthoformiat oder Alkohole wie n-Propanol, n-Butanol, Ethylenglykol, Diethylenglykol, Ethylenglykolmonomethylether, Cyclohexanol oder ähnliche Verbindungen verwendet. Bilden die verwendeten Ausgangsverbindungen bereits eine flüssige Mischung, kann auf ein zusätzliches Lösungsmittel verzichtet werden. Die Reaktionstemperaturen liegen bevorzugt zwischen 20 und 180°C, besonders bevorzugt zwischen 40 und 150°C. Der Druck ist bevorzugt normaler Atmosphärendruck. In Abhängigkeit von der Reaktivität der eingesetzten Verbindung IV ist die Verwendung eines Katalysators bzw. eines Katalysatorgemisches vorteilhaft. Als Katalysatoren eignen sich z.B. Ammoniumacetat, Piperidin und β-Alanin und deren Acetate.

Als Katalysatoren für die Umsetzung können bei sehr langen Reaktionszeiten zusätzlich Lewis-Säuren wie AlCl₃, ZrCl₄, TiCl₄ oder vor allem ZnCl₂ in den hierfür üblichen Mengen verwendet werden.

Die 2-Cyanacrylsäureester der Formel I, in denen r = ist, d.h., in denen ein Rest R¹ oder R² über ein Stickstoffatom an das β-C-Atom gebunden ist, lassen sich vorteilhaft herstellen, indem man einen Cyanessigsäureester der allgemeinen Formel III 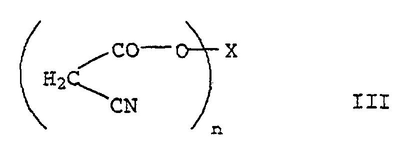 mit einem aromatischen Amin der Formel IVa 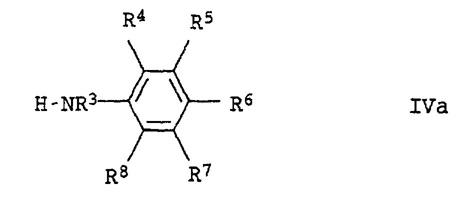 in Gegenwart von Trialkylorthoformiat umsetzt. Als Trialkylorthoformiate haben sich z.B. Trimethylorthoformiat und Triethylorthoformiat bewährt.

Die Cyanessigester III können beispielsweise durch Umsetzung von Cyanessigsäure oder deren Estern mit den entsprechenden Polyolen X(OH)ₙ in Gegenwart eines Katalysators wie Borsäure, Na₂CO₃ oder K₂CO₃ oder Tetrabutylorthotitanat vorzugsweise in Toluol oder Xylol hergestellt werden.

Die erfindungsgemäßen Verbindungen eignen sich in hervorragender Weise zum Stabilisieren von organischen Materialien gegen die Einwirkung von Licht, Sauerstoff und wärme.

Als Kunststoffe, die durch die erfindungsgemäßen Verbindungen I stabilisiert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);

Halogenhaltige Polymere, wie z.B. Polyvinylchlorid, Polyvinylfluorid, Polyvinylidenfluorid sowie deren Copolymere; Polymere, die sich von α,β-ungesättigten Säuren und deren Derivaten ableiten, wie Polyacrylate, Polymethacrylate, Polyacrylamide und Polyacrylnitrile;

Polymere, die sich von ungesättigten Alkoholen und Aminen bzw. von deren Acrylderivaten oder Acetalen ableiten, z.B. Polyvinylalkohol und Polyvinylacetat;

Polyurethane, Polyamide, Polyharnstoffe, Polyphenylenether, Polyester, Polycarbonate, Polyoxymethylene, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen I zum Stabilisieren von Polyolefinen, insbesondere von Polyethylen, von Polycarbonaten, von Polyamiden, von Polyestern, von Polystyrol, von ABS und von Polyurethanen verwendet. Insbesondere können auch Folien aus den genannten Kunststoffen stabilisiert werden.

Für diese Anwendungsbereiche werden die Verbindungen in Konzentrationen von 0,01 bis 5 Gew.-%, bezogen auf die Menge des Kunststoffs, eingesetzt, bevorzugt in einer Konzentration von 0,02 bis 2 Gew.-%. Die Kombination mit anderen Stabilisatoren, beispielsweise Antioxidantien, Metalldesaktivatoren oder anderen Lichtschutzmitteln sowie mit antistatischen oder flammhemmenden Mitteln, ist oft vorteilhaft. Besonders wichtige Costabilisatoren sind beispielsweise sterisch gehinderte Phenole sowie Phosphite, Phosphonite, Amine und Schwefelverbindungen.

Als geeignete Costabilisatoren kommen z.B. in Betracht:
Phenolische Antioxidationsmittel wie
2,6-Di-tert.-butyl-4-methylphenol,
   n-Octadecyl-β-(3,5-di-tert.-butyl-4-hydroxyphenol)-propionat,
   1,1,3-Tris-(2-methyl-4-hydroxy-5-tert.-butylphenyl)-butan,
   1,3,5-Trimethyl-2,4,6-tris-(3,5-di-tert.-butyl-4-hydroxybenzyl)-benzol,
   1,3,5-Tris-(3,5-di-tert.-butyl-4-hydroxybenzyl)-isocyanurat,
   1,3,5-Tris-[β-(3,5-di-tert.-butyl-4-hydroxyphenyl)-propionylethyl]-isocyanurat,
   1,3,5-Tris-(2,6-di-methyl-3-hydroxy-4-tert.-butylbenzyl) -isocyanurat und
   Pentaerythrit-tetrakis-[ß-(3,5-di-tert.-butyl-4-hydroxy)-propionat],
phosphorhaltige Antioxidantien wie
   Tris-(nonylphenyl)-phosphit, Distearylpentaerythritphosphit,
   Tris-(2,4-di-tert.-butyl-phenyl)-phosphit,
   Tris-(2-tert.-butyl-4-methylphenyl)-phosphit,
   Bis-(2,4-di-tert.-butylphenyl)-pentaerythritdiphosphit und
   Tetrakis-(2,4-di-tert.-butylphenyl)-4,4'-biphenylendiphosphit,
schwefelhaltige Antioxidantien wie
   Dilaurylthiodipropionat,
   Dimyristylthiodipropionat,
   Distearylthiodipropionat,
   Pentaerythrittetrakis-(β-laurylthiopropionat) und
   Pentaerythrittetrakis-(β-hexylthiopropionat),
   sterisch gehinderte Amine wie
   Bis-(2,2,6,6-tetramethylpiperidyl)-sebacat,
   Bis-(1,2,2,6,6-pentamethylpiperidyl)-sebacat,
   Bis-(1,2,2,6,6-pentamethylpiperidyl)-ester,
   N,N'-Bis(formyl)-bis (2,2,6,6-tetramethyl-4-piperidyl)-1,6-hexandiamin,
   das Kondensationsprodukt von
   1-Hydroxy-2,2,6,6-tetramethyl-4-hydroxypiperidin und Bernsteinsäure,
   das Kondensationsprodukt von
   N,N'-(2,2,6,6-Tetramethylpiperidyl)-hexamethylendiamin und
   4-tert.-Octylamino-2,6-dichlor,1,3,5-s-triazin,
   Poly-[3-(Eicosyl/Tetracosyl)-1-(2,2,6,6-tetramethylpiperidin-4-yl)-pyrrolidin-2,5-dion],
   Tris-(2,2,6,6-Tetramethylpiperidyl)-nitrilotriacetat, Tetrakis-(2,2,6,6-tetramethyl-4-piperidyl)-1,2,3,4-butantetracarbonsäure,
   1,1'-(1,2-Ethandiyl)-bis-(3,3,5,5-tetramethylpiperazinon),
   die Kondensationsprodukte von
   4-Amino-2,2,6,6-tetramethylpiperidinen und Tetramethylolacetylendiharnstoffen sowie
2-(2'-Hydroxyphenyl)-benztriazole,
   2-Hydroxybenzophenone,
   Arylester von Hydroxybenzoesäuren,
   α-Cyanozimtsäurederivate,
   Nickelverbindungen oder
   Oxalsäuredianilide.

Zur Vermischung der erfindungsgemäßen Verbindungen I, vor allem mit Kunststoffen, können alle bekannten Vorrichtungen und Methoden zum Einmischen von Stabilisierungsmitteln oder anderen Zusätzen in Polymere angewandt werden.

Die erfindungsgemäßen 2-Cyanacrylsäureester I zeichnen sich durch eine gute Verträglichkeit mit den üblichen Kunststoffarten und durch eine gute Löslichkeit und eine ausgezeichnete Verträglichkeit in den üblichen Lacksystemen aus. Sie haben in der Regel keine oder nur eine sehr geringe Eigenfarbe, sind bei den üblichen Kunststoff- und Lack-Verarbeitungstemperaturen stabil und nicht flüchtig und bewirken eine lange Schutzdauer der mit ihnen behandelten Materialien. Vor allem jedoch zeigen sie praktisch keine Migrationsneigung in Kunststoffen.

Die UV-Strahlung wird in drei Bereiche eingeteilt: den UV-A-Bereich (320-400 nm), den UV-B-Bereich (290-320 nm) und den UV-C-Bereich (200-290 nm). Der hochenergetische UV-C-Bereich wird überwiegend von der Ozonschicht absorbiert. Strahlung im UV-B-Bereich ist vor allem für die Entstehung von Sonnenbrand und Hautkrebs verantwortlich. Die UV-A-Strahlung bewirkt bei längerer Einwirkung sowohl die Hautbräunung, ist aber auch für die Alterung der Haut mitverantwortlich.

wegen der günstigen Löslichkeitseigenschaften sowie der guten Absorptionseigenschaften, besonders im UV-A-Bereich, eignen sich die erfindungsgemäßen Verbindungen besonders für Anwendungen im kosmetischen und dermatologischen Bereich. Auch zum Schutz kosmetischer Präparate wie Parfums, Cremes und Lotionen können die Verbindungen vorteilhaft eingesetzt werden. Besonders bevorzugt sind Kombinationen mit Lichtschutzmitteln, die im UV-B-Bereich absorbieren. Für kosmetische Formulierungen werden die 2-Cyanacrylsäureester I in Konzentrationen von 0,05 bis 15-Gew.-%, bevorzugt von 0,1 bis 10 Gew.-%, bezogen auf die Gesamtmenge der kosmetischen Formulierung, eingesetzt.

Weitere organische Materialien, denen die erfindungsgemäßen Verbindungen vorteilhaft zugemischt werden, sind Arzneimittelformulierungen wie Pillen und Zäpfchen, photographische Aufzeichnungsmaterialien, insbesondere photographische Emulsionen, sowie Vorprodukte für Kunststoffe und Lacke.

### Beispiele

### Herstellungsbeispiele

### Beispiel 1

16,2 g (0,04 mol) 2,2-Bis-(hydroxymethyl)-1,3-propandioltetracyanoacetat wurden in 100 ml N,N-Dimethylformamid (DMF) gelöst und auf 80°C erhitzt. Dazu tropfte man unter leichtem Stickstoffstrom 29,6 g (0,16 mol) Benzophenonimin (97 gew.-%ig), gelöst in 25 ml DMF, über 2 h zu. Bis zum Ende der Ammoniakentwicklung erwärmte man auf ca. 110°C. Danach kühlte man ab und setzte 300 ml Ethanol zu. Das Produkt wurde zunächst ölig und unter längerem Rühren fest. Man saugte ab und wusch mit Ethanol.

Man erhielt 37,5 g (88,4 %) der Theorie der Verbindung der Formel 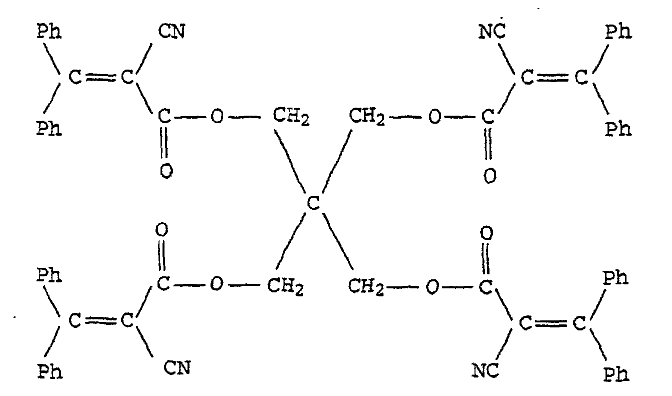 vom Schmelzpunkt 123 bis 126°C (glasartig); UV (CH₂Cl₂) :λₘₐₓ = 310 nm, ε = 50000.

### Beispiel 2

Die Verbindung der Formel 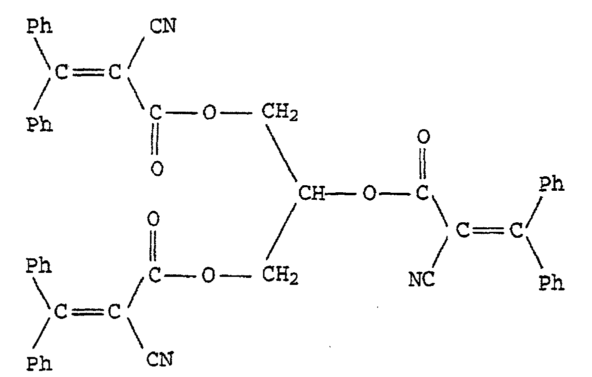 wurde in Analogie zu Beispiel 1 aus dem entsprechenden Cyanessigsäureester und Benzophenonimin hergestellt; Schmelzpunkt: 100 bis 104°C; UV (CH₂Cl₂) :λₘₐₓ = 310 nm, ε = 36400.

### Beispiel 3

Die Verbindung der Formel 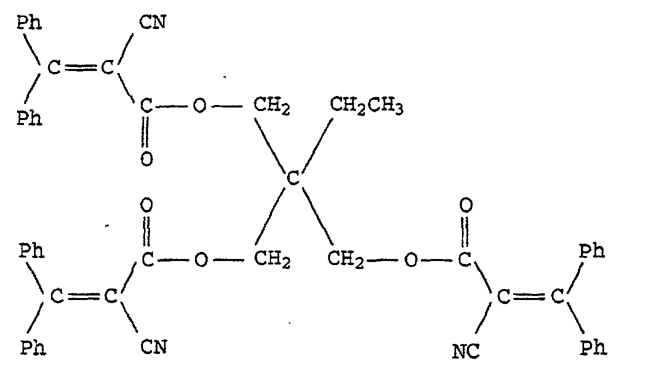 wurde in Analogie zu Beispiel 1 aus dem entsprechenden Cyansäureester und Benzophenonimin hergestellt; Schmelzpunkt: 92°C; UV (CH₂Cl₂) :λₘₐₓ = 308 nm, ε = 36700.

### Beispiel 4

Die Verbindung der Formel 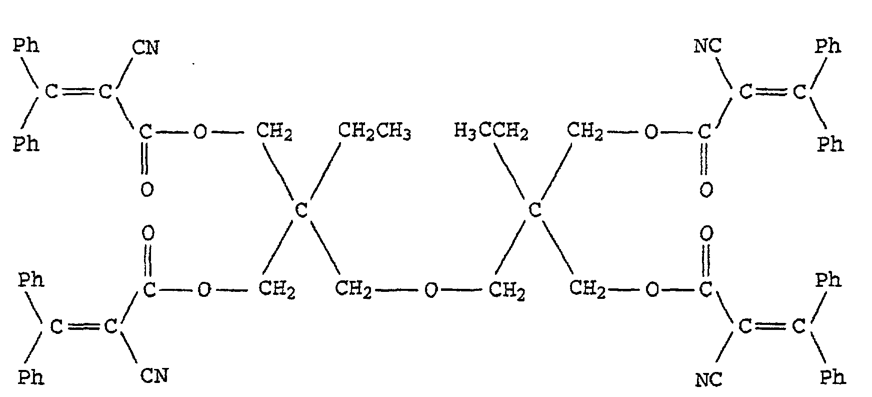 wurde in Analogie zu Beispiel 1 aus dem entsprechenden Cyanessigsäureester und Benzophenonimin hergestellt; Schmelzpunkt: 83 bis 95°C; UV (CH₂Cl₂) :λₘₐₓ = 308 nm, ε = 51700.

### Beispiel 5

Die Verbindung der Formel 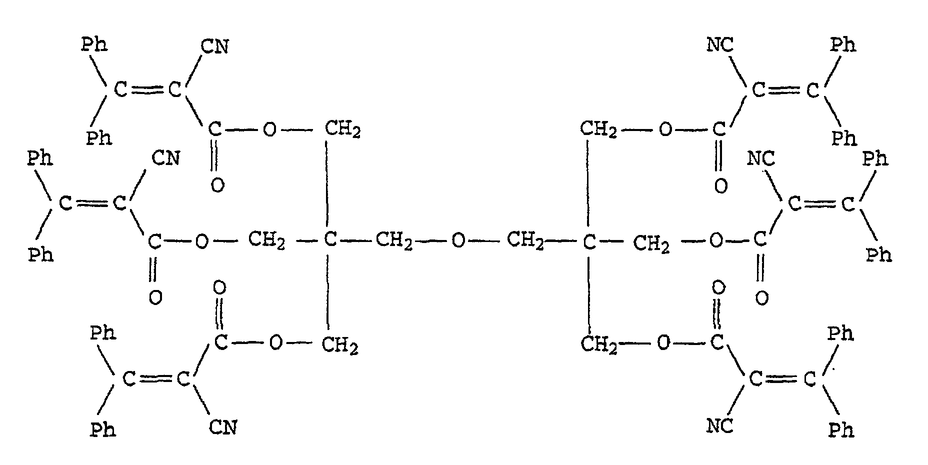 wurde in Analogie zu Beispiel 1 aus dem entsprechenden Cyanessigsäureester und Benzophenonimin hergestellt; Schmelzpunkt: 124 bis 128°C; UV (CH₂Cl₂) :λₘₐₓ = 308 nm, ε = 76000.

### Beispiel 6

30,3 g (0,075 mol) 2,2-Bis-(hydroxymethyl)-1,3-propandioltetracyanoacetat wurden mit 29,8 g (0,32 mol) Anilin und 52 g (0,35 mol) Trimethylorthoformiat 6 h unter Rückfluß erhitzt. Danach gab man 80 ml Ethanol zu und kochte die Suspension noch 1 h unter Rückfluß. Danach saugte man heiß ab und wusch den Rückstand gut mit Ethanol.

Man erhielt 55 g (90 % der Theorie) einer gelblichen Verbindung der Formel 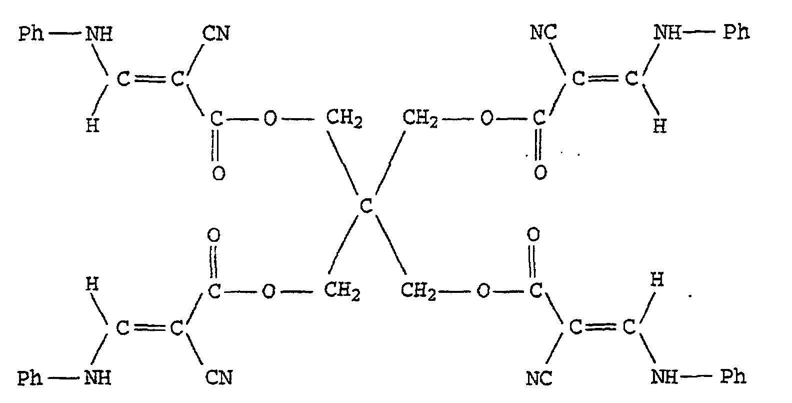 vom Schmelzpunkt 298 bis 300°C; UV (DMSO) :λₘₐₓ = 322 nm, ε = 98000 (DMSO = Dimethylsulfoxid).

### Beispiele 7 und 8

Die Verbindung der Formel 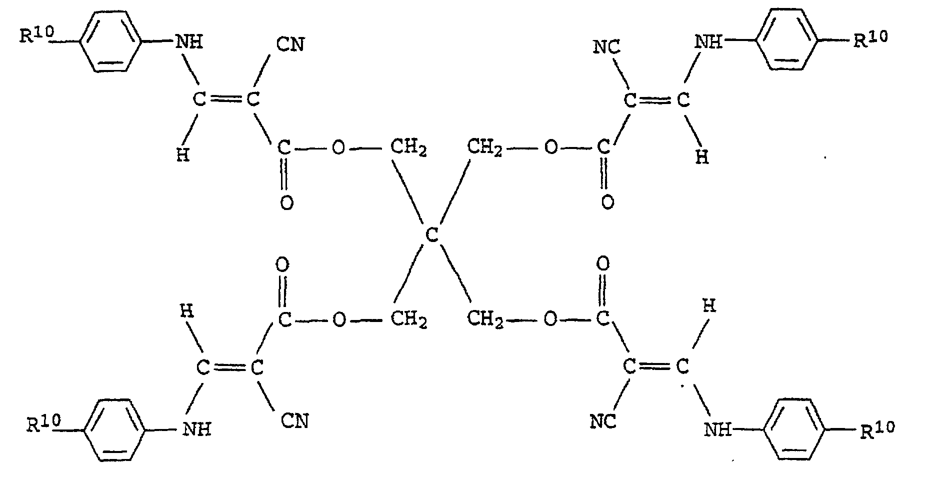 R¹⁰ = CH₃ (Beispiel 7) oder COOCH₂CH₃ (Beispiel 8)
wurden in Analogie zu Beispiel 6 aus dem entsprechenden Cyanessigsäureester, dem entsprechenden aromatischen Amin und Trimethylorthoformiat hergestellt; Schmelzpunkte: 321 bis 323°C (Beispiel 7) und 269 bis 273°C (Beispiel 8); UV (DMSO) :λₘₐₓ = 326 nm (Beispiel 7) und 334 nm (Beispiel 8), ε = 99000 (Beispiel 7) und 150000 (Beispiel 8).

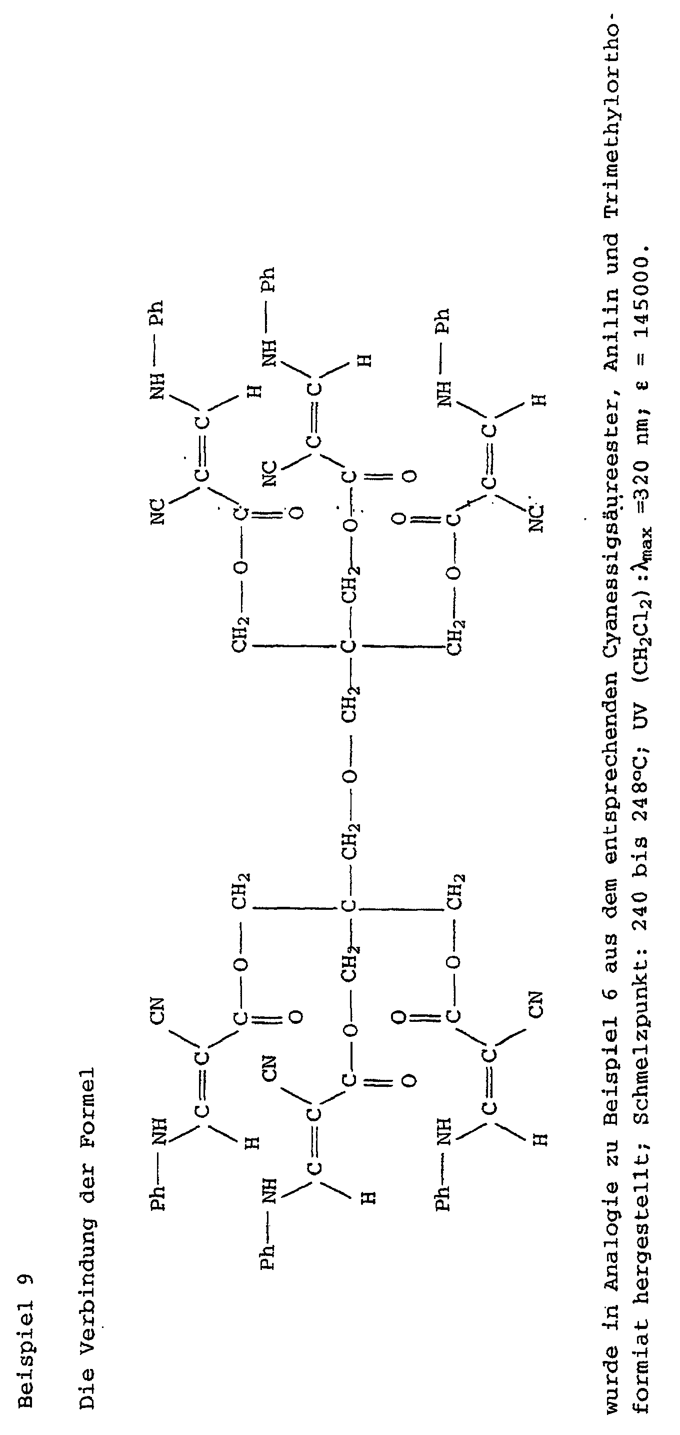

### Beispiele 10-33

Allgemeine Herstellvorschrift für die Umsetzung von Cyanessigsäureestern III mit Aldehyden (R¹ oder R² = Wasserstoff)
0,1 mol eines n-wertigen Cyanessigsäureesters III, 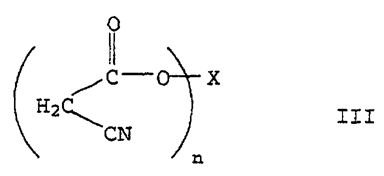 welcher durch Umsetzung von Cyanessigsäure mit dem entsprechenden n-wertigen Alkohol in bekannter Weise erhalten wurde,
wurden mit 0,12 n mol eines Aldehyds IVb 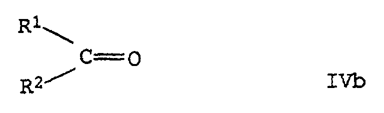 in 100 ml N,N-Dimethylacetammid in Gegenwart von 0,5 ml Piperidin und 0,3 ml Eisessig umgesetzt. Nach 3 Stunden bei 70°C wurde der Niederschlag abgetrennt, mit Methanol und Wasser gewaschen und getrocknet.

Die Einzelheiten dieser Versuche sowie die Eigenschaften der erhaltenen Verbindungen I sind der folgenden Tabelle zu entnehmen.

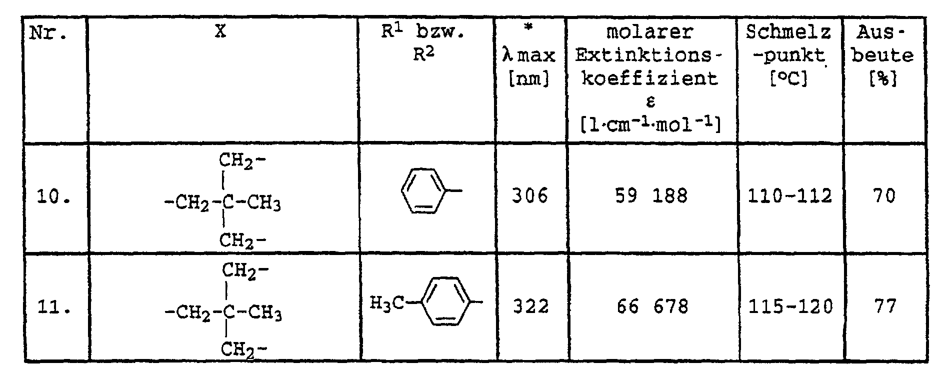

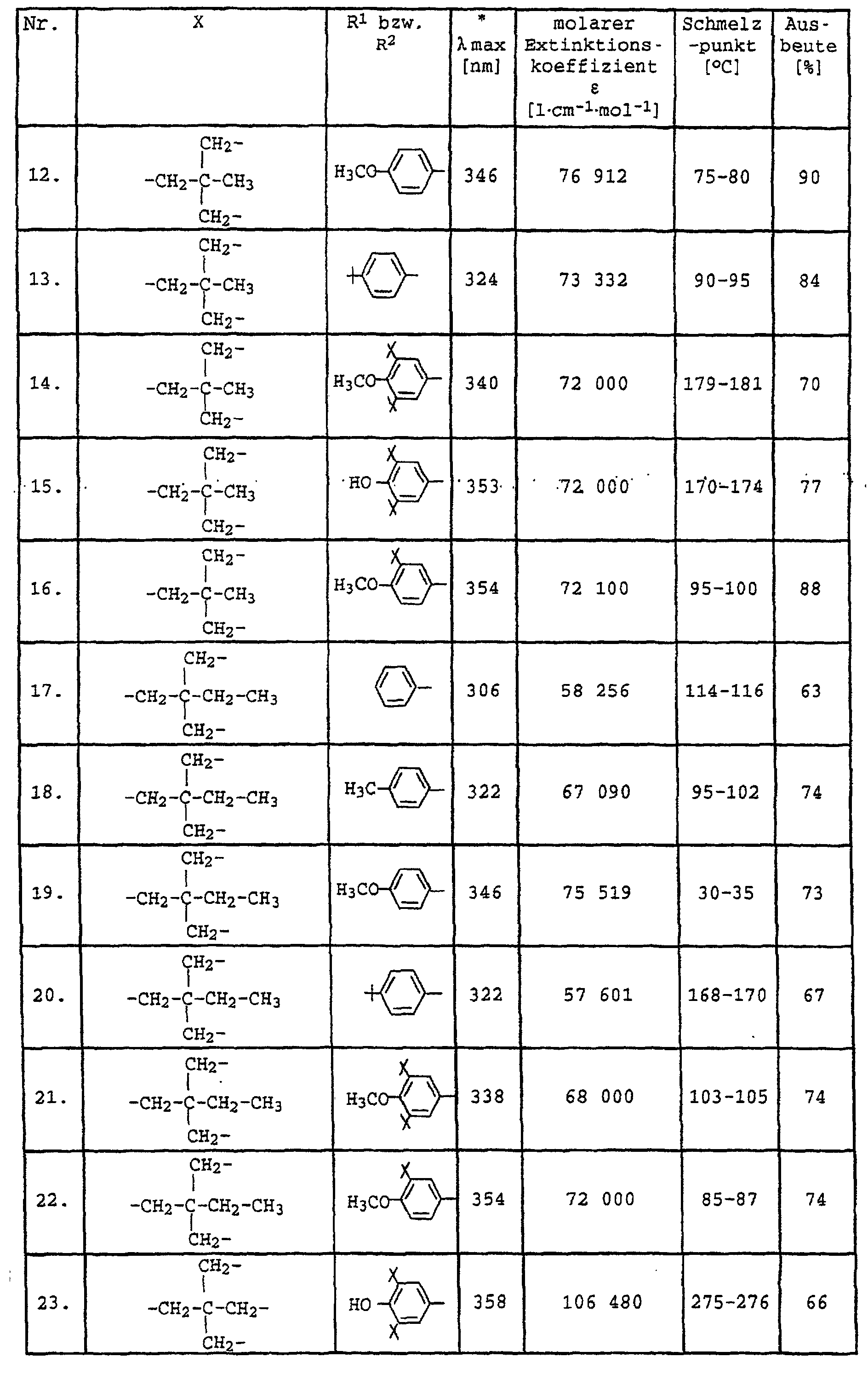

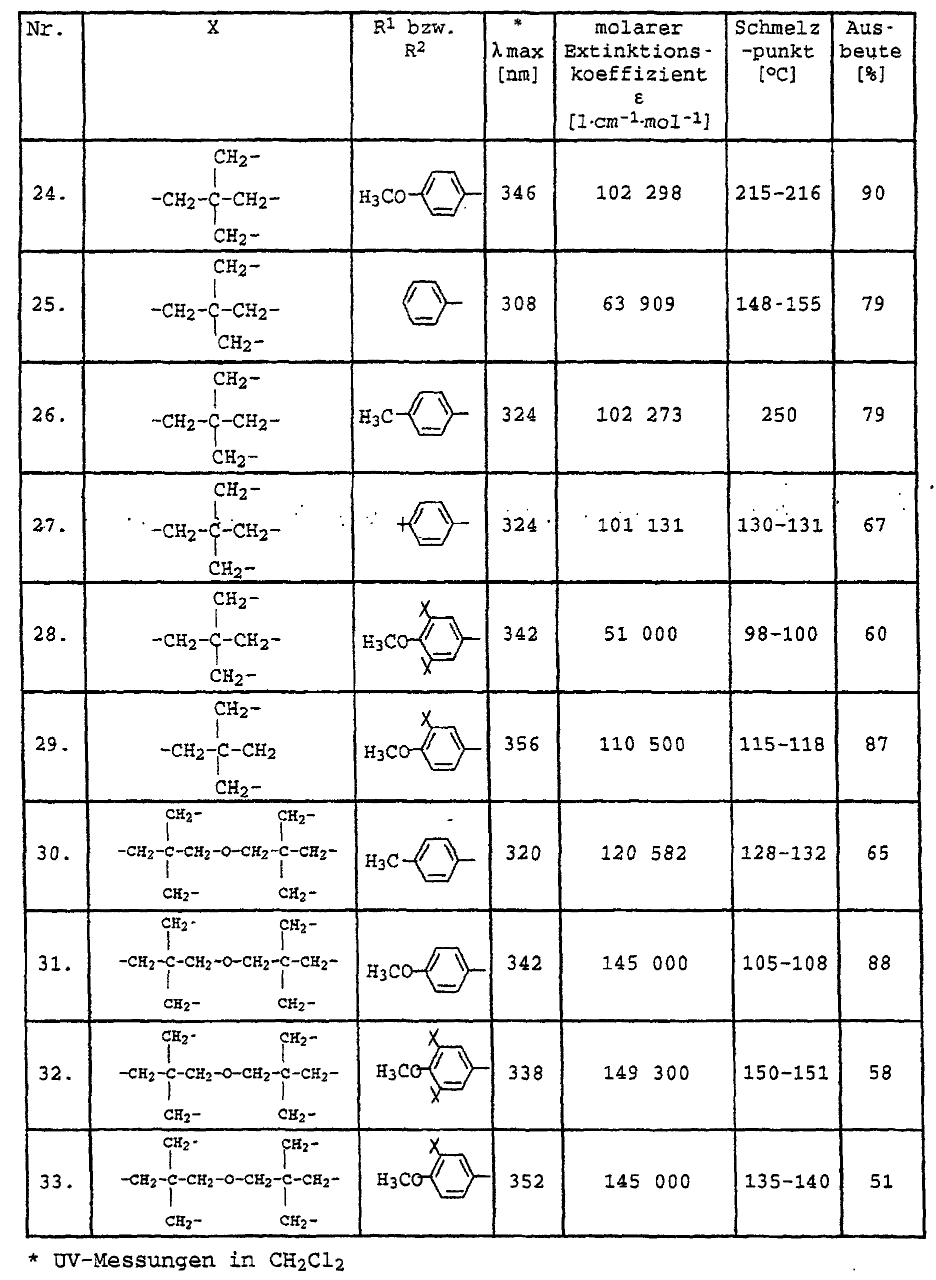

### Beispiel 34

### Anwendungsbeispiel: Migrationstest in Polyethylen

0,3 Gew.-% des nachfolgend angegebenen UV-Stabilisators wurden in Polyethylen durch zweimaliges Extrudieren bei 180°C Massetemperatur im Polymeren gelöst, danach wurde das Polymere granuliert und zu 100 µm dicken Folien geblasen.

Nach zehntägiger Lagerung bei Raumtemperatur (20°C) oder im Ofen (50°C) wurde die Oberfläche der Folie visuell nach folgenden Kriterien beurteilt:
- +: kein Belag
- o: geringer Belag
- -: starker Belag

Die nachfolgende Tabelle zeigt die verwendeten UV-Stabilisatoren und die Ergebnisse der Prüfungen:

| UV-Stabilisator | Lagerung bei 20°C | Lagerung bei 50°C |
|---|---|---|
| Verbindung aus Beispiel Nr. 1 | + | + |
| Verbindung A (zum Vergleich) | o | - |
| Verbindung B (zum Vergleich) | - | - |

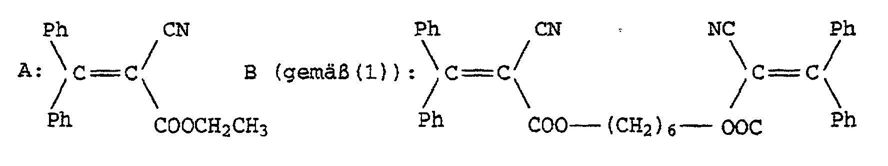

## Patentansprüche

1. 2-Cyanacrylsäureester der Formel I 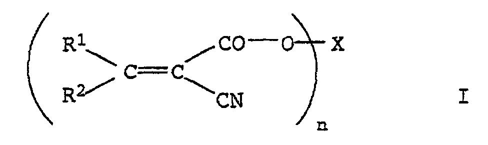 in der die Reste R¹ und R² Wasserstoff oder einen Rest mit einem iso- oder heterocyclischen Ringsystem mit mindestens einem iso- oder heteroaromatischen Kern bedeuten, wobei mindestens einer der Reste R¹ oder R² von Wasserstoff verschieden sein muß,
n einen Wert von > 2 bis 10 hat und
X den Rest eines n-wertigen aliphatischen oder cycloaliphatischen Polyols mit 3 - 20 C-Atomen bezeichnet, wobei ein cycloaliphatischer Rest auch 1 bis 2 Heteroatome enthalten kann und ein aliphatischer Rest durch bis zu 8 nicht benachbarte Sauerstoffatome, Schwefelatome, Imino- oder C₁-C₄-Alkyliminogruppen unterbrochen sein kann.

2. 2-Cyanacrylsäureester nach Anspruch 1, in denen einer der Reste R¹ und R² einen Rest der Formel II 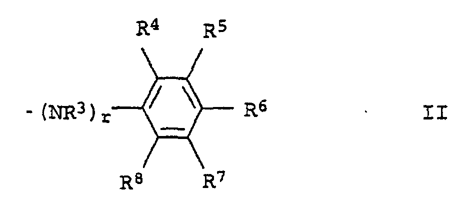 bedeutet, worin R³ Wasserstoff oder C₁-C₁₀-Alkyl bedeutet, r für die Zahl 0 oder 1 steht und R⁴ bis R⁸ unabhängig voneinander Wasserstoff, C₁-C₈-Alkyl, Chlor, Brom, Cyano, Nitro, Amino, Mono (C₁-C₄-alkyl)amino, Di(C₁-C₄-alkyl)amino, Hydroxy, C₁-C₈-Acyl, C₁-C₈-Acyloxy, C₁-C₁₈-Alkoxy, C₁-C₁₂-Alkoxycarbonyl, C₃-C₆-Cycloalkyl oder C₃-C₆-Cycloalkoxycarbonyl bezeichnen.

3. 2-Cyanacrylsäureester nach Anspruch 2, in denen R³ Wasserstoff, Methyl oder Ethyl bedeutet.

4. 2-Cyanacrylsäureester nach Anspruch 2, in denen bis zu drei der Reste R⁴ bis R⁸ Wasserstoff, C₁-C₄-Alkyl, Chlor, Cyano, Hydroxy, Acetyl, C₁-C₅-Alkoxy, C₁-C₈-Alkoxycarbonyl oder Cyclohexoxycarbonyl und die übrigen dieser Reste Wasserstoff bedeuten.

5. 2-Cyanacrylsäureester nach den Ansprüchen 2 bis 4, in denen R⁶ eine Hydroxygruppe oder eine C₁-C₄-Alkoxygruppe bedeutet.

6. 2-Cyanacrylsäureester nach den Ansprüchen 2 bis 5, in denen R⁵ und/oder R⁷ Wasserstoff, Methyl oder tert.-Butyl bedeuten.

7. 2-Cyanacrylsäureester nach den Ansprüchen 2 bis 6, in denen r=O ist.

8. 2-Cyanacrylsäureester nach den Ansprüchen 1 bis 7, in denen X den Rest eines n-wertigen Polyols mit 3 bis 12 C-Atomen bedeutet, welcher in seinem linearen oder verzweigten Kohlenstoffgerüst durch bis zu 3 nichtbenachbarte Sauerstoffatome unterbrochen sein kann und n für eine Zahl von 3 bis 6 steht.

9. Verfahren zur Herstellung von 2-Cyanacrylsäureestern gemäß den Ansprüchen 1 bis 8, in denen r = O ist, **dadurch gekennzeichnet, daß** man einen Cyanessigsäureester der allgemeinen Formel III 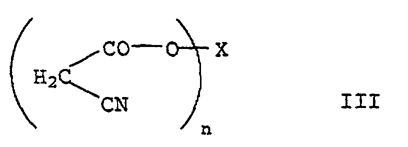 mit n mol einer Verbindung der allgemeinen Formel IV 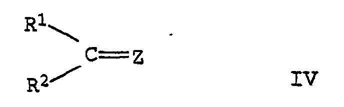 in der Z Sauerstoff oder NH bedeutet, unter den Bedingungen der Knoevenagel-Kondensation in einem polaren Lösungsmittel und in Gegenwart eines Katalysators umsetzt.

10. Verfahren zur Herstellung von 2-Cyanacrylsäureestern gemäß den Ansprüchen 2 bis 6 und 8, in denen r = 1 ist, **dadurch gekennzeichnet, daß** man einen Cyanessigsäureester der allgemeinen Formel III 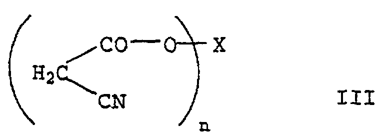 mit einem aromatischen Amin der Formel IVa 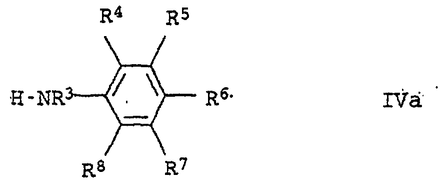 in Gegenwart von Trialkylorthoformiat umsetzt.

11. Verwendung der 2-Cyanacrylsäureester gemäß den Ansprüchen 1 bis 8 als Lichtschutzmittel oder Stabilisatoren für organische Materialien.

12. Verwendung der 2-Cyanacrylsäureester gemäß den Ansprüchen 1 bis 8 als Lichtschutzmittel oder Stabilisatoren in kosmetischen oder dermatologischen Zubereitungen.

13. Verwendung der 2-Cyanacrylsäureester gemäß den Ansprüchen 1 bis 8 als Lichtschutzmittel oder Stabilisatoren in Kunststoffen oder Lacken.

14. Gegen die Einwirkung von Licht, Sauerstoff und Wärme stabilisierte organische Materialien, welche 0,01 bis 10 Gew.-%, bezogen auf die Menge des organischen Materials, eines oder mehrerer 2-Cyanacrylsäureester gemäß den Ansprüchen 1 bis 8 enthalten.

15. Gegen die Einwirkung von Licht, Sauerstoff und Wärme stabilisierte kosmetische oder dermatologische Zubereitungen, welche 0,01 bis 15 Gew.-%, bezogen auf die Menge dieser Zubereitungen, eines oder mehrerer 2-Cyanacrylsäureester gemäß den Ansprüchen 1 bis 8 enthalten.

16. Gegen die Einwirkung von Licht, Sauerstoff und Wärme stabilisierte Kunststoffe und Lacke, welche 0,01 bis 10 Gew.-%, bezogen auf die Menge des Kunststoffs oder Lacks, eines oder mehrerer 2-Cyanacrylsäureester gemäß den Ansprüchen 1 bis 8 enthalten.

## Claims

1. A 2-cyanoacrylic ester of the formula I 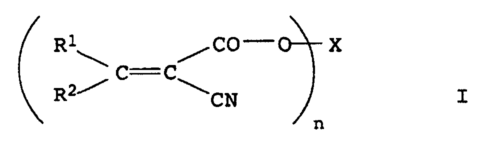 where R¹ and R² are each hydrogen or a radical having an iso- or heterocyclic ring system with at least one iso- or heteroaromatic nucleus, and at least one of the radicals R¹ or R² must be different from hydrogen,
n is from > 2 to 10, and
X is the radical of an n-hydric aliphatic or cycloaliphatic polyol having 3-20 carbon atoms, it also being possible for a cycloaliphatic radical to contain 1 or 2
hetero atoms, and for an aliphatic radical to be interrupted by up to 8 non-adjacent oxygen atoms, sulfur atoms, imino or C₁-C₄-alkylimino groups.

2. A 2-cyanoacrylic ester as claimed in claim 1, wherein one of the radicals R¹ and R² is a radical of the formula II 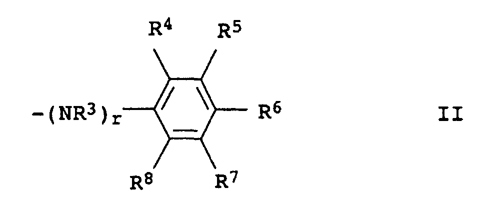 where R³ is hydrogen or C₁-C₁₀-alkyl, r is 0 or 1, and R⁴ to R⁸ are each, independently of one another, hydrogen, C₁-C₈-alkyl, chlorine, bromine, cyano, nitro, amino, mono-(C₁-C₄-alkyl)amino, di(C₁-C₄-alkyl)amino, hydroxyl, C₁-C₈-acyl, C₁-C₈-acyloxy, C₁-C₁₈-alkoxy, C₁-C₁₂-alkoxycarbonyl, C₃-C₆-cycloalkyl or C₃-C₆-cycloalkoxycarbonyl.

3. A 2-cyanoacrylic ester as claimed in claim 2, wherein R³ is hydrogen, methyl or ethyl.

4. A 2-cyanoacrylic ester as claimed in claim 2, wherein up to three of the radicals R⁴ to R⁸ are hydrogen, C₁-C₄-alkyl, chlorine, cyano, hydroxyl, acetyl, C₁-C₅-alkoxy, C₁-C₈-alkoxycarbonyl or cyclohexoxycarbonyl, and the remainder of these radicals are hydrogen.

5. A 2-cyanoacrylic ester as claimed in claims 2 to 4, wherein R⁶ is a hydroxyl group or a C₁-C₄-alkoxy group.

6. A 2-cyanoacrylic ester as claimed in claims 2 to 5, wherein R⁵ and/or R⁷ are hydrogen, methyl or tert-butyl.

7. A 2-cyanoacrylic ester as claimed in claims 2 to 6, wherein r is 0.

8. A 2-cyanoacrylic ester as claimed in claims 1 to 7, wherein X is the radical of an n-hydric polyol having 3 to 12 carbon atoms, which may be interrupted in its linear or branched carbon skeleton by up to 3 non-adjacent oxygen atoms, and n is from 3 to 6.

9. A process for preparing 2-cyanoacrylic esters as claimed in claims 1 to 8, where r is 0, which comprises reacting a cyanoacetic ester of the formula III 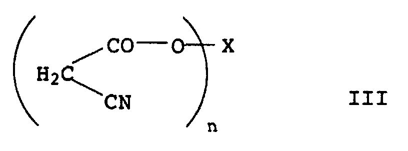 with n mol of a compound of the formula IV 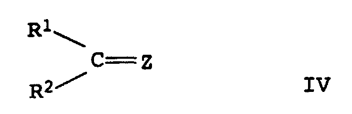 where Z is oxygen or NH, under the conditions of the Knoevenagel condensation in a polar solvent and in the presence of a catalyst.

10. A process for preparing 2-cyanoacrylic esters as claimed in claims 2 to 6 and 8, where r is 1, which comprises reacting a cyanoacetic ester of the formula III 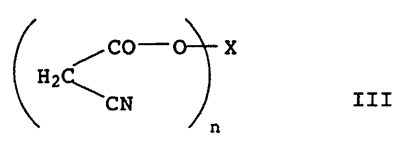 with an aromatic amine of the formula IVa 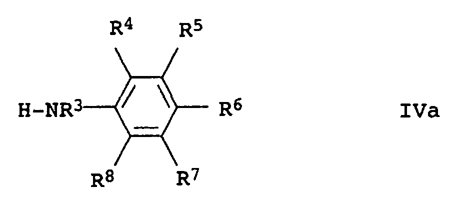 in the presence of trialkyl orthoformate.

11. The use of the 2-cyanoacrylic esters as claimed in claims 1 to 8 as stabilizers, in particular against the action of light, for organic materials.

12. The use of the 2-cyanoacrylic esters as claimed in claims 1 to 8 as stabilizers, in particular against the action of light, in cosmetic or dermatological preparations.

13. The use of the 2-cyanoacrylic esters as claimed in claims 1 to 8 as stabilizers, in particular against the action of light, in plastics or paints.

14. Organic material stabilized against the action of light, oxygen and heat, which comprises from 0.01 to 10% by weight, based on the amount of organic material, of one or more 2-cyanoacrylic esters as claimed in claims 1 to 8.

15. A cosmetic or dermatological preparation stabilized against the action of light, oxygen and heat, which comprises from 0.01 to 15% by weight, based on the amount of this preparation, of one or more 2-cyanoacrylic esters as claimed in claims 1 to 8.

16. A plastic or paint stabilized against the action of light, oxygen and heat, which comprises from 0.01 to 10% by weight, based on the amount of the plastic or paint, of one or more 2-cyanoacrylic esters as claimed in claims 1 to 8.

## Revendications

1. Ester de l'acide 2-cyanoacrylique de formule I 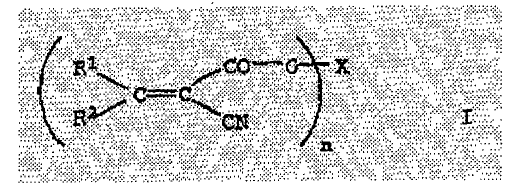 dans laquelle les résidus R¹ et R² représentent un atome d'hydrogène ou un résidu ayant un système cyclique isocyclique ou hétérocyclique possédant au moins un noyau isoaromatique ou hétéroaromatique, où au moins un des résidus R¹ ou R² est obligatoirement différent d'un atome d'hydrogène,
n vaut > 2 à 10, et
X représente un résidu polyol aliphatique ou cycloaliphatique de valence n et ayant 3 à 20 atomes de C, où un résidu cycloaliphatique peut contenir éventuellement 1 à 2 hétéroatomes, et un résidu aliphatique peut être interrompu par jusqu'à 8 atomes d'oxygène ou de souffre ou groupes imino ou alkylimino en C₁ à C₄ non vicinaux.

2. Esters de l'acide 2-cyanoacrylique selon la revendication 1, dans lesquels un des résidus R¹ et R² représente un résidu de formule II 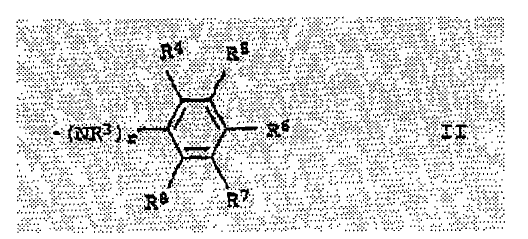 dans laquelle R³ représente un atome d'hydrogène ou un groupe alkyle en C₁ à C₁₀, r représente le nombre 0 ou 1, et R⁴ à R⁸ représentent, indépendamment l'un de l'autre, un atome d'hydrogène, un groupe alkyle en C₁ à C₈, un atome de chlore, de brome, un groupe cyano, nitro, amino, mono(alkyle en C₁ à C₄)amino, di(alkyle en C₁ à C₄)amino, hydroxy, acyle en C₁ à C₈, acyloxy en C₁ à C₈, alcoxy en C₁ à C₁₈, alcoxy-carbonyle en C₁ à C₁₂, cycloalkyle en C₃ à C₆ ou cycloalcoxy-carbonyle en C₃ à C₆.

3. Esters de l'acide 2-cyanoacrylique selon la revendication 2, dans lesquels R³ représente un atome d'hydrogène ou un groupe méthyle ou éthyle.

4. Esters de l'acide 2-cyanoacrylique selon la revendication 2, dans lesquels jusqu'à trois résidus parmi les résidus R⁴ à R⁸ représentent un atome d'hydrogène, un groupe alkyle en C₁ à C₄, un atome de chlore, un groupe cyano, hydroxy, acétyle, alcoxy en C₁ à C₅, alcoxy-carbonyle en C₁ à C₈ ou cyclohexoxy-carbonyle, et les autres de ces résidus représentent un atome d'hydrogène.

5. Esters de l'acide 2-cyanoacrylique selon les revendications 2 à 4, dans lesquels R⁶ représente un groupe hydroxy ou un groupe alcoxy en C₁ à C₄.

6. Esters de l'acide 2-cyanoacrylique selon les revendications 2 à 5, dans lesquels R⁵ et/ou R⁷ représentent un atome d'hydrogène, un groupe méthyle ou tert-butyle.

7. Esters de l'acide 2-cyanoacrylique selon les revendications 2 à 6, dans lesquels r = 0.

8. Esters de l'acide 2-cyanoacrylique selon les revendications 1 à 7, dans lesquels X représente un résidu polyol de valence n ayant 3 à 12 atomes de C qui peut être interrompu au niveau de son squelette carboné linéaire ou ramifié par jusqu'à 3 atomes d'oxygène non vicinaux, et n représente un nombre compris dans la plage de 3 à 6.

9. Procédé de préparation d'esters de l'acide 2-cyanoacrylique selon les revendications 1 à 8, dans lesquels r = 0, **caractérisé en ce que** l'on met à réagir un ester de l'acide cyanoacétique de formule générale III 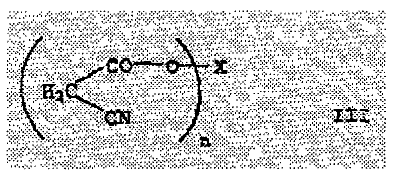 avec n moles d'un composé de formule générale IV 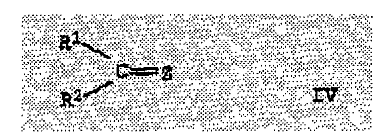 dans laquelle Z représente un atome d'oxygène ou NH, dans les conditions d'une condensation de Knoevenagel, dans un solvant polaire et en présence d'un catalyseur.

10. Procédé de préparation d'esters de l'acide 2-cyanoacrylique selon les revendications 2 à 6 et 8, dans lesquels r = 1, **caractérisé en ce que** l'on met à réagir un ester de l'acide cyanoacétique de formule générale III 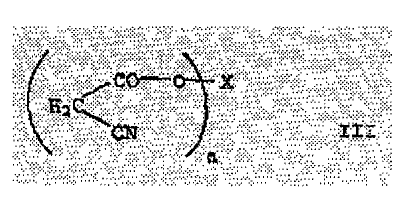 avec une amine aromatique de formule IVa 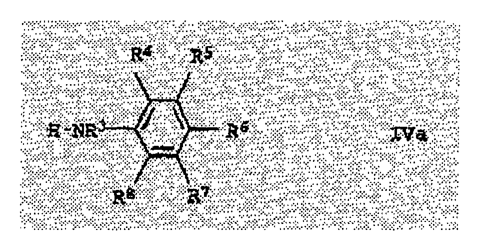 en présence du trialkylorthoformiate.

11. Mise en oeuvre des esters de l'acide 2-cyanoacrylique selon les revendications 1 à 8 en tant qu'agent stabilisant contre l'effet de la lumière ou en tant que stabilisant, pour les matières organiques.

12. Mise en oeuvre des esters de l'acide 2-cyanoacrylique selon les revendications 1 à 8 en tant qu'agent stabilisant contre l'effet de la lumière ou en tant que stabilisant, dans les préparations cosmétiques ou dermatologiques.

13. Mise en oeuvre des esters de l'acide 2-cyanoacrylique selon les revendications 1 à 8 en tant qu'agent stabilisant contre l'effet de la lumière ou en tant que stabilisant, dans les matières synthétiques ou les laques.

14. Les matières organiques stabilisées contre les effets de la lumière, de l'oxygène et de la chaleur qui contiennent 0,01% à 10% en poids, d'un ou de plusieurs des esters de l'acide 2-cyanoacrylique selon les revendications 1 à 8, par rapport à la quantité de matière organique.

15. Les préparations cosmétiques ou dermatologiques stabilisées contre les effets de la lumière, de l'oxygène et de la chaleur qui contiennent 0,01% à 15% en poids, d'un ou de plusieurs des esters de l'acide 2-cyanoacrylique selon les revendications 1 à 8, par rapport à la quantité de ces préparations.

16. Les matières synthétiques ou les laques stabilisées contre les effets de la lumière, de l'oxygène et de la chaleur qui contiennent 0,01% à 10% en poids, d'un ou de plusieurs des esters de l'acide 2-cyanoacrylique selon les revendications 1 à 8, par rapport à la quantité de matière synthétique ou de laque.
